# EUROPÄISCHE PATENTSCHRIFT

(11) **EP 2 026 705 B1**
(45) Veröffentlichungstag und Bekanntmachung des Hinweises auf die Patenterteilung: **20.02.2013**
(21) Anmeldenummer: 07726009.9
(22) Anmeldetag: 14.06.2007
(51) Int. Cl.: A61B 17/29

(54) **CHIRURGISCHE GREIFZANGE**
SURGICAL GRIPPING FORCEPS
PINCE À SÉQUESTRE CHIRURGICALE

(30) Priorität: 14.06.2006 DE 102006028001
(43) Veröffentlichungstag der Anmeldung: 25.02.2009
(73) Patentinhaber: Karl Storz GmbH & Co. KG, 78532 Tuttlingen (DE)
(72) Erfinder: REINAUER, Josef, 72488 Sigmaringen (DE); GANTER, Hans, 78532 Tuttlingen (DE)
(86) Internationale Anmeldenummer: PCT/EP2007/005245
(87) Internationale Veröffentlichungsnummer: WO 2007/144172

(56) Entgegenhaltungen:
- WO-A-02/07627
- WO-A-02/064020
- DE-U1- 20 120 249
- US-A- 5 275 615
- US-A- 5 290 309
- US-A- 5 569 299
- US-A- 6 083 150
- US-B1- 6 818 007

## Beschreibung

Die Erfindung betrifft eine chirurgische Greifzange gemäß den Merkmalen des Oberbegriffs des Anspruchs 1.

Eine solche Greifzange ist z. B. aus EP 1 211 989 B1 bekannt. Wenn die beiden Greifbacken, das Schubelement und eine Schubstange in zwei Halbschalen zusammengebaut sind, wird eine Hülse über die beiden Halbschalen geschoben, um die gesamte Baugruppe zusammenzuhalten und um eine radiale Bewegung der Bauteile in den Halbschalen zu unterbinden. Insgesamt benötigt diese Greifzange eine Vielzahl von Bauteilen und ist zudem schwer zu montieren.

Aus der US 5,342,390 ist eine weitere Greifzange bekannt. Die Greifbacken dieser Zange sind auf einem gemeinsam genutzten Schwenkzapfen gelagert. Der Schwenkzapfen schneidet die Mittellinie des Zangengrundkörpers. Dadurch können die an den Greifbacken angeformten Hebelarme, über die Greifbacken bewegt werden, zwangsläufig nur relativ kurz ausgebildet werden. Auch wirkt auf jeden Hebelarm der Greifbacken jeweils ein separates Schubelement.

Der vorliegenden Erfindung liegt die Aufgabe zugrunde, eine chirurgische Greifzange zu entwickeln, die bei einer üblichen Zangenbetätigungskraft zwischen den Greifbacken eine große Zangenklemmkraft ermöglicht. Hierbei soll bei kleiner Zangenbaugröße eine geringe Bauteileanzahl realisiert werden und zudem eine einfache Montage der Greifzange möglich sein.

Diese Aufgabe wird mit den Merkmalen des Anspruchs 1 gelöst. Die laparoskopische Chirurgie benötigt ein spezielles Instrumentarium. Allen Instrumenten gemeinsam ist die Miniaturisierung, weshalb die laparoskopische Chirurgie auch endoskopische Mikrochirurgie genannt wird. Die Instrumente werden durch lange Hülsen, die einen Durchmesser haben, der in der Regel zwischen vier bis zwölf Millimetern liegt, in das Abdomen meist über Torkarhülsen eingeführt und außerhalb der Bauchhöhle mit Handkraft bedient.

In der Mikrochirurgie müssen sowohl feine Greifinstrumente für die Präparation als auch grobe für die Extraktion resezierter Organe vorhanden sein. Verschiedene Greifinstrumente mit einem Durchmesser von drei, fünf oder zehn Millimetern stehen zur Verfügung. Es gibt auf der einen Seite atraumatische und auf der anderen gezahnte Pinzetten. Sie haben Greifbacken, die spitz oder breit, fein oder grob sind. Teilweise verfügen die Greifinstrumente auch über Arretiermechanismen.

Die Greifzangen müssen einfach und sicher zu bedienen sein. Dazu gehört u.a. eine große Handkraftübersetzung durch den Greifzangenmechanismus. Besonders vorteilhaft ist es auch, wenn die Greifzange, also der Teil, der im Abdomen vorn aus der Torkarhülse herausschaut, möglichst wenige Teile hat. Wenige Teile haben immer auch wenige Bewegungsfugen. Dies senkt das Verletzungsrisiko und erleichtert das Desinfizieren der Greifzange. Letzteres gilt nur, sofern es sich bei der entsprechenden Zange nicht um ein Einweggerät handelt.

Weitere Einzelheiten der Erfindung ergeben sich aus den Unteransprüchen und der nachfolgenden Beschreibung einer schematisch dargestellten Ausführungsform:
- Figur 1:: Greifzange, geschlossen; in dimetrischer Ansicht;
- Figur 2:: Greifzange, geöffnet; von vorn dargestellt;
- Figur 3:: wie Figur 2, jedoch von hinten dargestellt;
- Figur 4:: Greifzange, geschlossen; in der Draufsicht;
- Figur 5:: Prinzipskizze zu halber Greifzange, geschlossen;
- Figur 6:: wie Figur 5, jedoch offen;
- Figur 7:: Prinzipskizze zu ganzer Greifzange, geschlossen;
- Figur 8:: wie Figur 7, jedoch offen;
- Figur 9:: Greifzange mit nur einer Greifbacke, geschlossen;
- Figur 10:: wie Figur 9, jedoch halb offen;
- Figur 11:: wie Figur 9, jedoch ganz offen; Figur 12:_Grundkörper in dimetrischer Ansicht;
- Figur 13:: Schubelement in dimetrischer Ansicht;
- Figur 14:: Greifbacke in dimetrischer Ansicht.

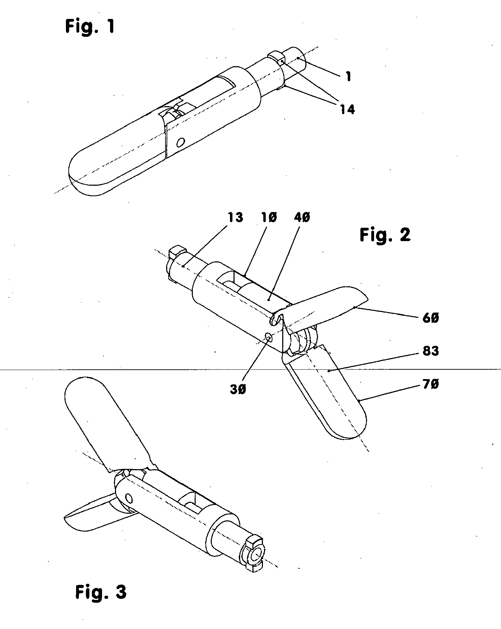

Die Figuren 1 - 3 zeigen eine chirurgische Greifzange in geschlossener und geöffneter Darstellung. Die Greifzange umfasst einen Grundkörper 10, ein Schubelement 40, zwei Greifbacken 60, 70 und einen Führungsbolzen 30.

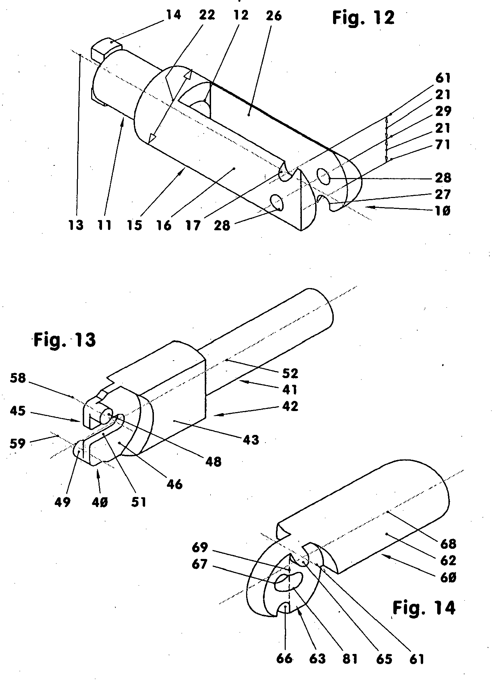

Der Grundkörper 10, vgl. auch Figur 12, besteht aus einem Rohr- 11 und einem Gabelabschnitt 15. Der Rohrabschnitt 11 hat eine zentrale Bohrung 12, in der das Schubelement 40 geführt ist. An seinem hinteren Ende sind zwei einander gegenüberliegende Adapterelemente 14 angeformt. Über diese Adapterelemente 14 wird der Grundkörper 10 an einem nicht dargestellten Gehäuserohr lösbar, z.B. mittels eines Bajonettverschlusses, befestigt.

Der Gabelabschnitt 15 hat zwei Gabelarme 16, 26, die am Rohrabschnitt 11 angeordnet sind. Die Außenwandung der Gabelarme 16, 26 sind beispielsweise Teile eines Zylindermantels. Der Durchmesser dieses Zylinders ist der Grundkörperdurchmesser 22. Er beträgt beim Ausführungsbeispiel 4,8 mm. Die Innenwandungen der Gabelarme 16, 26 sind Ebenen, die einander parallel gegenüber liegen. Der Abstand der Ebenen entspricht z.B. dem Innendurchmesser der zentralen Bohrung 12. An ihren vorderen Enden haben die Gabelarme 16, 26 jeweils eine Bohrung 28. Die zueinander fluchtenden Bohrungen 28 haben eine Mittellinie 29, die sich mit der Mittellinie 13 des Grundkörpers 10 senkrecht schneidet.

Der nach Figur 12 vordere Gabelarm 16 hat oberhalb der Bohrung 28 eine z.B. u-förmige, nutartige Ausnehmung 17. Der Ausnehmungsgrund hat bereichsweise die Fläche eines Zylindermantels. Das Zentrum des Zylindermantels ist eine obere Schwenkachse 61. Der hintere Gabelarm 26 hat eine vergleichbare Ausnehmung 27. Letztere ist hier nach unten orientiert und umgibt bereichsweise eine untere Schwenkachse 71. Die Schwenkachsen 61, 71 und die Mittellinie 29 der Bohrungen 28 liegen in einer Ebene. Diese Ebene ist normal zur Mittellinie 13 des Grundkörpers 10 ausgerichtet.

Figur 13 zeigt das Schubelement 40. Es besteht aus einem Schubzapfenabschnitt 41, einem Führungsabschnitt 42 und einem Lagerabschnitt 45. Der Schubzapfenabschnitt 41, über den das Schubelement 40 im Grundkörper 10 gelagert ist, hat eine zylindrische Form. An seinem freien Ende trägt er ggf. eine Gewindebohrung. In letzterer wird dann die im Gehäuserohr der Zange geführte Betätigungsstange lösbar befestigt. Die Gewindebohrung, das Gehäuserohr und die Betätigungsstange sind in den Figuren nicht dargestellt.

An den Schubzapfenabschnitt 41 schließt sich der Führungsabschnitt 42 an. Letzterer hat zumindest annähernd die Form eines Quaders mit zwei planen, zueinander parallelen Seitenflächen 43. Diese Seitenflächen 43 liegen bei der montierten Greifzange an den Innenwandungen der Gabelarme 16, 26 des Grundkörpers 10 an. Dort haben sie u.a. die Funktion einer Verdrehsicherung. Die oben und unten an die Seitenflächen 43 angrenzenden gekrümmten Zylinderteilflächen gehören zu einem Zylinder, dessen Durchmesser dem Grundkörperdurchmesser entspricht.

Der Führungsabschnitt 42 geht in einen Lagerabschnitt 45 über. Der Lagerabschnitt 45 entspricht einer dünnwandigen Platte, die zwei Gelenkzapfen 48, 49 und eine Führungsnut 51 aufweist. Die Gelenkzapfen 48, 49 haben zueinander parallele Mittellinien 58, 59. Beide Mittellinien 58, 59 spannen eine Ebene auf, die normal zur Mittellinie 52 des Schubelements 40 liegt. Der nach Figur 13 obere Gelenkzapfen 48 ist nach vorn orientiert, während der untere 49 nach hinten ausgerichtet ist. Beide Gelenkzapfen 48, 49 haben gegenüber der Mittellinie 52 den gleichen Abstand. Der Abstand zwischen den Mittellinien 58, 59 beträgt mehr als 2/3 des Grundkörperdurchmessers 22. Die Gelenkzapfen 48, 49 haben z.B. einen Durchmesser von 1 mm.

Mittig zwischen den Gelenkzapfen 48, 49 befindet sich eine gerade Führungsnut 51, die zum freien Ende des Lagerabschnitts 45 hin offen ist. Das geschlossene Ende der Führungsnut 51 hat eine teilzylindrische Ausrundung. Die Mittellinie der Ausrundung schneidet die Mittellinie 52 des Bauteils 40 senkrecht.

In Figur 14 ist eine von zwei Greifbacken 60 dargestellt. Im räumlichen Verhältnis zu den in den Figur 12 und 13 dargestellten Bauteilen 10 und 40 ist es die obere Greifbacke. Die Greifbacke 60 besteht aus einem Backenabschnitt 62 und einem Schwenkbereichsabschnitt 63. Der Backenabschnitt 62 hat die Form der Hälfte eines längsgeteilten Zylinders. Der Durchmesser dieses Zylinders stimmt mit dem Grundkörperdurchmesser 22 überein. Das vordere freie Ende des Backenabschnitts 62 ist abgerundet. Der Abrundungsradius entspricht dem halben Grundkörperdurchmesser 22.

Der zumindest annähernd kreisscheibenförmige Schwenkbereichsabschnitt 63 erstreckt sich in der vorderen Hälfte der Greifbacke 60. D.h. die hintere plane Fläche des Schwenkbereichsabschnitts 63 sie kontaktiert eine Lagerfläche 46 des Lagerabschnitts 45 des Schubelements 40 - liegt in einer Ebene, die von der Bauteilmittenebene um die halbe Breite des Lagerabschnitts 45 entfernt ist. In dieser Mittenebene verläuft die Bauteilmittellinie 68. Auf letzterer liegt auch die hier ebene, atraumatische Backengreiffläche 83, vgl. Figur 2.

Im oberen Bereich hat der Schwenkbereichsabschnitt 63 einen sich nach vorn erstreckenden Schwenkzapfen 65. Der Schwenkzapfen 65, der eine Mittellinie 61 aufweist, hat zumindest nach unten hin eine zylindrische Außenkontur. Unterhalb des Schwenkzapfens 65 befindet sich eine sichelförmige Führungsausnehmung 67. Der Krümmungsradius der Führungsausnehmung 67 hat einen Mittelpunkt, der auf der Mittellinie 61 des Schwenkzapfens 65 liegt. Folglich verfügt die Führungsausnehmung 67 über mindestens eine kreisbogenförmige Kante 81, deren Mittelpunkt ebenfalls auf der Mittellinie 61 liegt. Unterhalb der Führungsausnehmung 67 ist in den Schwenkbereichsabschnitt 63 von unten her eine Kulissenausnehmung 66 eingearbeitet. Die Kulissenausnehmung 66 ist beispielsweise eine gerade Nut, deren Nutbreite geringfügig größer ist als der Durchmesser der Gelenkzapfen 48, 49 des Schubelements 40. Der Ausnehmungsgrund hat auch hier bereichsweise die Fläche eines Zylindermantels.

In Figur 14 ist auf der sichtbaren planen Fläche, die bei montierter Greifzange an der Innenwandung des entsprechenden Gabelarms 16, 26 des Grundkörpers 10 anliegt, eine gestrichelte Hilfslinie 69 eingezeichnet. Die Hilfslinie 69 steht zudem senkrecht auf der Ebene der Backengreiffläche 83. An dieser Hilfslinie 69 liegen die vorderste Mantellinie des Schwenkzapfens 65 und die Kante der hinteren, ebenen Wandung der Kulissenausnehmung 66 an.

Das fünfte und letzte Bauteil der Greifzange ist der zylindrische Führungsbolzen 30, vgl. Figur 2. Sein Durchmesser beträgt beim Ausführungsbeispiel 1 mm. Seine Länge ist geringfügig kleiner als der Grundkörperdurchmesser 22. Er sitzt in den Bohrungen 28 der Greifarme 16, 26 beispielsweise mittels eines Querpresssitzes.

Alle fünf Teile 10, 30, 40, 60, 70 der Greifzange sind beispielsweise jeweils aus einem rost- und säurebeständigen Stahl, z.B. dem Chromstahl X20Cr13 gefertigt.

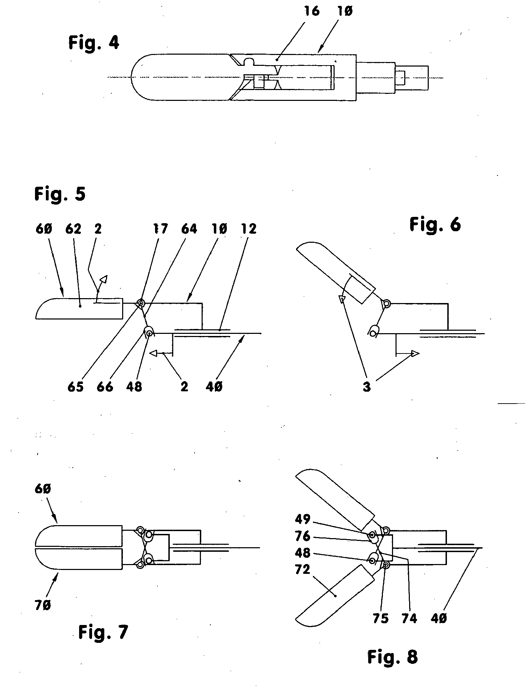

Bevor das Zusammenwirken der Zangenteile erläutert wird, wird kurz auf das Funktionsprinzip eingegangen. In den Figuren 5 und 6 ist das Funktionsprinzip zunächst für nur eine Greifbacke 60 dargestellt.

Nach Figur 5 ist die Greifbacke 60 ein Backenabschnitt 62, an dem ein Hebelarm 64 mit einer Kulissenausnehmung 66 und einem Schwenkzapfen 65 befestigt ist. Der Grundkörper 10 ist eine Geradführung 12 mit einem daran angeordneten Schwenkzapfenlager 17. In der Geradführung 12 ist das Schubelement 40 gelagert. Es greift über einen Gelenkzapfen 48 in die Kulissenausnehmung 66 ein.

Zum Öffnen des Greifbackens 60 wird das Schubelement 40 in die Geradführung 12 nach links verschoben. Der Gelenkzapfen 48 wirkt auf die Kulissenführung 66. Diese schwenkt zusammen mit dem Hebelarm 64 und dem Backenabschnitt 62 im Uhrzeigersinn nach oben. Hierbei dreht sich der Schwenkzapfen 65 im Schwenkzapfenlager 17 des Grundkörpers 10, vgl. Figur 6.

In den Figuren 7 und 8 ist das Prinzip für die gesamte Greifzange dargestellt. Dazu werden die in den Figuren 5 und 6 dargestellten Skizzen zunächst nach unten gespiegelt. Anschlie-βend werden Original und Spiegelbild solange ineinandergeschoben, bis nach Figur 4 die Greifbacken 60, 70 aneinander liegen. Um nun hier mit einer Geradführung 12 auszukommen, werden beide Gelenkzapfen 48, 49 auf einem gemeinsamen Schubelement 40 fest angeordnet.

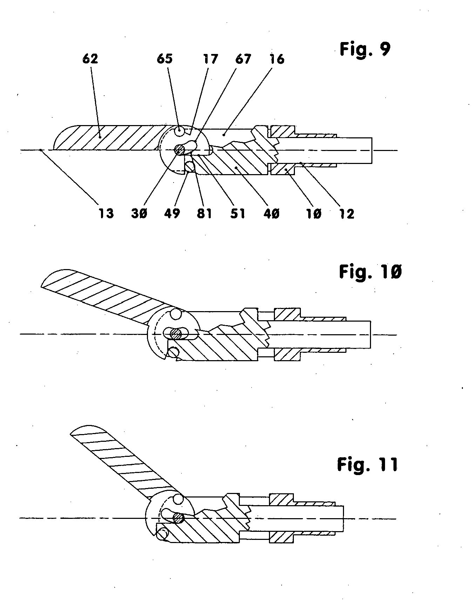

Die Einzelteile 10, 30, 40, 60, 70 der realen Greifzange, vgl. Figuren 12 bis 14, werden montiert, indem das Schubelement 40 mit seinem Führungsabschnitt 42 voraus fast vollständig in die Bohrung 12 des Grundkörpers 10 geschoben wird. Die Einstecktiefe ist in Figur 9 dargestellt. Nun wird die obere Greifbacke 60 von oben her in den Spalt zwischen dem vorderen Gabelarm 16 und der Lagerfläche 46 des Schubelements 40 eingelegt. Die Greifbacke 60 ist hierbei in Strecklage, d.h. ihre Mittellinie 68 verläuft während des Einsteckvorganges parallel zur Mittellinie 13 des Grundkörpers 10. Bei der Abwärtsbewegung kommt der Schwenkzapfen 65 der Greifbacke 60 in der Ausnehmung 17 des Grundkörpers 10 zur Anlage. Zeitgleich wird die Kulissenausnehmung 66 der Greifbacke 60 über den Gelenkzapfen 59 des Schubelements 40 geschoben. Das Einlegen der Greifbacke 60 ist beendet, sobald sich die Mittellinie 68 der Greifbacke 60 mit der Mittellinie 13 des Grundkörpers 10 deckt. Die untere Greifbacke 70 wird in vergleichbarer Weise von unten her eingelegt.

Bei dieser Bauteilanordnung liegen im Gelenkbereich der Greifzange fünf Ausnehmungen bzw. Bohrungen zumindest bereichsweise übereinander. Dies sind - von außen nach innen betrachtet - die beiden Bohrungen 28 der Gabelarme 16, 26, die beiden sichelförmigen Führungsausnehmungen 67, 77 der Greifbacken 60, 70 und die Führungsnut 51 des Schubelements 40. Durch alle Ausnehmungen hindurch wird abschließend der Führungsbolzen 30 gesteckt und fixiert, vgl. Figuren 1 bis 4.

Die bisher nicht beschriebene Funktion der sichelförmigen Ausnehmung 67, der Greifbacke 60 wird in den Figuren 9 bis 11 verdeutlicht. In diesen Figuren ist die Greifzange im Längsschnitt ohne die untere Greifbacke 70 dargestellt. Aus dem Schubelement 40 ist zudem ein Stück ausgebrochen.

Der Grundkörper 10 lagert die Greifbacke 60 in der Ausnehmung 17 und auf dem Führungsbolzen 30. In der einseitig offenen Ausnehmung 17 liegt der Schwenkzapfen 65. Die sichelförmige Führungsausnehmung 67 umgreift den Führungsbolzen 30. Um nun ein unbeabsichtigtes Auswandern des Schwenkzapfens 65 bzw. der Greifbacke 67 nach oben zu verhindern, liegt die Führungsausnehmung 66 an dem Führungsbolzen 30 zumindest über die Kante 81 an. Durch die Anlage der Führungsausnehmung 67 am Führungsbolzen 30 ist es möglich, die Schwenkachse 61 der Greifbacke 60 innerhalb des Grundkörpers 10 weit von der Mittellinie 13 entfernt zu legen. Dies erlaubt einen großen Greifzangenhebelarm.

### Bezugszeichenliste

- 1: Längsachse bzw.,Mittellinie der Greifzange
- 2: Öffnungsrichtung
- 3: Schließrichtung

- 10: Grundkörper
- 11: Rohrabschnitt
- 12: Bohrung, zentral; Geradführung
- 13: Mittellinie von (10)
- 14: Adapterelemente
- 15: Gabelabschnitt
- 16: Gabelarm
- 17: Ausnehmung, Schwenkzapfenlager

- 21: Schwenkachsenabstand
- 22: Grundkörperdurchmesser

- 26: Gabelarm
- 27: Ausnehmung, Schwenkzapfenlager
- 28: Bohrungen für Führungsbolzen
- 29: Mittellinie

- 30: Führungsbolzen

- 40: Schubelement
- 41: Schubzapfenabschnitt
- 42: Führungsabschnitt
- 43: Seitenflächen, plan
- 45: Lagerabschnitt
- 46: Lagerflächen
- 48: Gelenkzapfen
- 49: Gelenkzapfen
- 51: Führungsnut, gerade
- 52: Mittellinie zu (40)
- 58: Mittellinie zu (48)
- 59: Mittellinie zu (49)

- 60: erste Greifbacke
- 61: Schwenkachse

- 62: Backenabschnitt
- 63: Schwenkbereichsabschnitt
- 64: Hebelarm
- 65: Schwenkzapfen
- 66: Kulissenausnehmung
- 67: Führungsausnehmung, sichelförmig
- 68: Mittellinie
- 69: Hilfslinie

- 70: zweite Greifbacke
- 71: Schwenkachse
- 72: Backenabschnitt
- 74: Hebelarm
- 75: Schwenkzapfen
- 76: Kulissenausnehmung
- 77: Führungsausnehmung, sichelförmig

- 81: Kante
- 83: Backengreiffläche

## Patentansprüche

1. Chirurgische Greifzange mit zwei gegenüber einem Grundkörper (10) beweglichen Greifbacken (60, 70), wobei jede Greifbacke (60, 70) eine eigene gegenüber dem Grundkörper (10) ortsfeste Schwenkachse und einen Hebelarm (64, 74) hat und wobei die Hebelarme (64, 74) für ein beidseitiges Verschwenken an ein Schubelement (40) angelenkt sind,
**dadurch gekennzeichnet,**
- **dass** der Grundkörper (10) einen vorderen Gabelabschnitt (11) mit zwei Gabelarmen (16, 26) aufweist,
- **dass** die Schwenkachsen (61, 71) der Greifbacken (60, 70) jeweils durch einen greifbackenseitigen Schwenkzapfen (65, 75) und eine grundkörperseitige Ausnehmung (17, 27) gebildet sind, wobei die grundkörperseitige Ausnehmung (17, 27) jeweils eine nutförmige Ausnehmung in jedem der beiden Gabelarme (16, 26) ist, in welcher jeweils einer der greifbackenseitigen Schwenkzapfen (65, 75) liegt,
- **dass** jede Greifbacke (60, 70) eine sichelförmige Führungsausnehmung (67) aufweist, durch welche ein im Grundkörper (10) gelagerter Führungsbolzen (30) geführt ist, und
- **dass** die einzelne Schwenkachse (61, 71) von der Mittellinie (13) des Grundkörpers (10) mindestens einen Abstand (21) hat, der größer als 38% der maximalen Grundkörperbreite oder des maximalen Grundkörperdurchmessers (22) ist.

2. Chirurgische Greifzange gemäß Anspruch 1,
**dadurch gekennzeichnet, dass** im Grundkörper (10) ein Schubelement (40) beide Greifbacken (60, 70) anlenkt.

3. Chirurgische Greifzange gemäß Anspruch 1,
**dadurch gekennzeichnet, dass** das Schubelement (40) im Grundkörper (10) mindestens zweifach geführt ist.

4. Chirurgische Greifzange gemäß Anspruch 1,
**dadurch gekennzeichnet, dass** die Schwenkachsen (61, 71) der Greifbacken (60, 70) parallel zueinander angeordnet sind.

5. Chirurgische Greifzange gemäß Anspruch 1,
**dadurch gekennzeichnet, dass** die Schwenkachsen (61, 71) der Greifbacken (60, 70) in einer Ebene liegen, die normal zur Mittellinie (13) des Grundkörpers (10) orientiert ist.

6. Chirurgische Greifzange gemäß einem der Ansprüche 1 bis **5,**
**dadarch gekennzeichnet**, dass die grundkörperseitige Ausnehmung (17, 27) den greifbackenseitigen Schwenkzapfen (65, 75) nur teilweise umschließt.

7. Chirurgische Greifzange gemäß einem der Ansprüche 1 bis 6,
**dadurch gekennzeichnet, dass** die Führungsausnehmung (67, 77) mindestens eine gekrümmte Kante (81) hat, die einen Kreisbogen um die Schwenkachse (61, 71) des Schwenkzapfens (65, 75) bildet.

8. **Chirurgische Greifzange gemäß Anspruch 1,**
**dadurch gekennzeichnet, dass** der Grundkörper (10) und das Schubelement (40) getriebetechnisch ein Schiebepaar bilden, wobei das Schubelement (40) im Grundkörper (10) verdrehsicher gelagert ist.

9. **Chirurgische Greifzange gemäß Anspruch 1,**
**dadurch gekennzeichnet, dass** der Grundkörper (10), das Schubelement (40) und die Greifbacken (60, 70) getriebetechnisch zwei schwingende Kurbelschleifen bilden, wobei die einzelne Kulissenausnehmung (67, 77) der Kurbelschleifen jeweils an einem Hebelarm (64, 74) der jeweiligen Greifbacke (60, 70) angeordnet ist.

## Claims

1. Surgical gripping forceps with two gripping jaws (60, 70) movable relative to a main body (10), each gripping jaw (60, 70) having its own pivot axis, stationary relative to the main body (10), and a lever arm (64, 74), and the lever arms (64, 74) being articulated on a push element (40) for pivoting to both sides, **characterized in that**
- the main body (10) has a front fork portion (11) with two fork arms (16, 26),
- the pivot axes (61, 71) of the gripping jaws (60, 70) are each formed by a pivot pin (65, 75) on the gripping jaws and by a recess (17, 27) on the main body, each recess (17, 27) on the main body being a groove-shaped recess which is formed in each of the two fork arms (16, 26) and in each of which lies one of the pivot pins (65, 75) on the gripping jaws,
- each gripping jaw (60, 70) has a sickle-shaped guide recess (67) through which a guide pin (30) mounted in the main body (10) is guided,
and
- the individual pivot axis (61, 71) is at a distance (21) from the midline (13) of the main body (10) that is at least greater than 38% of the maximum width of the main body or of the maximum diameter (22) of the main body.

2. Surgical gripping forceps according to Claim 1, **characterized in that**, in the main body (10), a push element (40) links both gripping jaws (60, 70).

3. Surgical gripping forceps according to Claim 1, **characterized in that** the push element (40) is guided at least two-fold in the main body (10).

4. Surgical gripping forceps according to Claim 1, **characterized in that** the pivot axes (61, 71) of the gripping jaws (60, 70) are arranged parallel to each other.

5. Surgical gripping forceps according to Claim 1, **characterized in that** the pivot axes (61, 71) of the gripping jaws (60, 70) lie in a plane that is oriented perpendicularly with respect to the midline (13) of the main body (10).

6. Surgical gripping forceps according to one of Claims 1 to 5, **characterized in that** the recess (17, 27) on the main body only partially encloses the pivot pin (65, 75) on the gripping jaws.

7. Surgical gripping forceps according to one of Claims 1 to 6, **characterized in that** the guide recess (67, 77) has at least one curved edge (81) that forms an arc of a circle around the pivot axis (61, 71) of the pivot pin (65, 75).

8. Surgical gripping forceps according to Claim 1, **characterized in that** the main body (10) and the push element (40) form a slide pairing mechanism, the push element (40) being mounted in the main body (10) in a manner secure against rotation.

9. Surgical gripping forceps according to Claim 1, **characterized in that** the main body (10), the push element (40) and the gripping jaws (60, 70) form two oscillating slider crank mechanisms, the individual crank recess (67, 77) of the slider cranks being arranged in each case on a lever arm (64, 74) of the respective gripping jaw (60, 70).

## Revendications

1. Pince chirurgicale de saisie présentant deux mâchoires de saisie (60, 70) qui peuvent être déplacées par rapport à un corps de base (10),
chaque mâchoire de saisie (60, 70) présentant son propre axe de pivotement fixe par rapport au corps de base (10) et un bras de levier (64, 74),
les bras de levier (64, 74) étant articulés sur un élément de poussée (40) de manière à permettre un pivotement bilatéral,
**caractérisée en ce que**
le corps de base (10) présente une partie avant de fourche (11) dotée de deux bras de fourche (16, 26),
**en ce que** les axes de pivotement (61, 71) des mâchoires de saisie (60, 70) sont tous deux formés par un tourillon de pivotement (65, 75) situé du côté de la mâchoire de saisie et d'une découpe (17, 27) située du côté du corps de base,
la découpe (17, 27) située du côté du corps de base étant une découpe en rainure formée dans chacun des deux bras de fourche (16, 26) et dans laquelle est situé un des tourillons de pivotement (65, 75) situés du côté de la mâchoire de saisie,
**en ce que** chaque mâchoire de saisie (60, 70) présente une découpe de guidage (67) en forme de croissant traversée par un tourillon de guidage (30) monté dans le corps de base (10) et
**en ce que** les différents axes de pivotement (61, 71) présentent par rapport à la ligne centrale (13) du corps de base (10) une distance (21) qui est supérieure à au moins 38 % de la largeur maximale du corps de base ou du diamètre maximum (22) du corps de base.

2. Pince chirurgicale de saisie selon la revendication 1, **caractérisée en ce qu'**un élément de poussée (40) articule les deux mâchoires de saisie (60, 70) dans le corps de base (10).

3. Pince chirurgicale de saisie selon la revendication 1, **caractérisée en ce que** l'élément de poussée (40) est guidé au moins deux fois dans le corps de base (10).

4. Pince chirurgicale de saisie selon la revendication 1, **caractérisée en ce que** les axes de pivotement (61, 71) des mâchoires de saisie (60, 70) sont parallèles l'un à l'autre.

5. Pince chirurgicale de saisie selon la revendication 1, **caractérisée en ce que** les axes de pivotement (61, 71) des mâchoires de saisie (60, 70) sont situés dans un plan orienté perpendiculaire à la ligne centrale (13) du corps de base (10).

6. Pince chirurgicale de saisie selon l'une des revendications 1 à 5, **caractérisée en ce que** la découpe (17, 27) située du côté du corps de base n'entoure que partiellement le tourillon de pivotement (65, 75) situé du côté des mâchoires de saisie.

7. Pince chirurgicale de saisie selon l'une des revendications 1 à 6, **caractérisée en ce que** la découpe de guidage (67, 77) présente au moins une arête courbe (81) qui forme un arc de cercle autour de l'axe de pivotement (61, 71) du tourillon de pivotement (65, 75).

8. Pince chirurgicale de saisie selon la revendication 1, **caractérisée en ce que** le corps de base (10) et l'élément de poussée (40) forment une paire coulissante de transmission, l'élément de poussée (40) étant monté dans le corps de base (10) de manière à ne pas pouvoir tourner.

9. Pince chirurgicale de saisie selon la revendication 1, **caractérisée en ce que** le corps de base (10), l'élément de poussée (40) et les mâchoires de saisie (60, 70) forment deux boucles pivotantes de manivelle de transmission, les différentes découpes de coulisse (67, 77) des boucles de manivelle étant disposées chacune sur un bras de levier (64, 74) de la mâchoire de saisie (60, 70) respective.
